(19) Europäisches Patentamt
European Patent Office
Office européen des brevets

(11) Veröffentlichungsnummer: **0 207 956**
**B1**

(12) # EUROPÄISCHE PATENTSCHRIFT

(45) Veröffentlichungstag der Patentschrift: **04.07.90**

(21) Anmeldenummer: **86900124.8**

(22) Anmeldetag: **12.12.85**

(86) Internationale Anmeldenummer:
**PCT/EP85/00701**

(87) Internationale Veröffentlichungsnummer:
**WO 86/03493 19.06.86 Gazette 86/13**

(51) Int. Cl.[5]: **C 07 K 1/00**, C 07 K 7/10, A 61 K 37/02

(54) **HIRUDIN-PA UND DESSEN DERIVATE, VERFAHREN ZU DEREN HERSTELLUNG UND DEREN VERWENDUNG.**

(30) Priorität: **13.12.84 DE 3445532**

(43) Veröffentlichungstag der Anmeldung:
**14.01.87 Patentblatt 87/03**

(45) Bekanntmachung des Hinweises auf die Patenterteilung:
**04.07.90 Patentblatt 90/27**

(84) Benannte Vertragsstaaten:
**AT BE CH DE FR GB IT LI LU NL SE**

(56) Entgegenhaltungen:
**EP-A-0 142 860**

**Chemical Abstracts, Band 85, 1976, Columbus, Ohio (US) T.E. Petersen et al.: "Primary structure of hirudin, a thrombin-specific inhibitor", siehe S. 234, Zusammenfassung 105932p & Protides Biol.Fluids.Proc. Colloq.1975(Publ. 1976)23,145-9(Eng.)**

**Die Akte enthält technische Angaben, die nach dem Eingang der Anmeldung eingereicht wurden und die nicht in dieser Patentschrift enthalten sind.**

(73) Patentinhaber: **UCP GEN-PHARMA AG**
**Solothurnstrasse 24**
**CH-3422 Kirchberg (CH)**

(72) Erfinder: **DODT, Johannes**
**Aberlestrasse 4**
**D-8000 München 70 (DE)**
Erfinder: **SEEMÜLLER, Ursula**
**Destouches-Str. 60**
**D-8000 München 40 (DE)**
Erfinder: **FRITZ, Hans**
**Neulinger Str. 15**
**D-8011 Hohenbrunn (DE)**
Erfinder: **FINK, Ernst**
**Stelhorner Str. 1**
**D-2910 Westerstede-Giesselhorst (DE)**

(74) Vertreter: **Kinzebach, Werner, Dr. et al**
**Patentanwälte Reitstötter, Kinzebach und Partner Sternwartstrasse 4 Postfach 86 06 49**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 207 956 B1

Courier Press, Leamington Spa, England.

(56) Entgegenhaltungen:
Chemical Abstracts, Band 100, 1984, Columbus, Ohio (US) J.Y. Chang: "The functional domain of hirudin,a thrombin-specific inhibitor", siehe Seite 227, Zusammenfassung 81858u & FEBS Lett. 1983, 164(2), 307-13(Eng).

Chemical Abstracts, Band 100, 1984, Columbus, Ohio (US) J.Dodt et al.: "The complete amino acid sequence of hirudin, a thrombin specific inhibitor. Application of color carboxymethylation", siehe S. 227,

Zusammenfassung 152937g, & FEBS Lett. 1984, 165(2), 180-4(Eng.)

## Beschreibung

Die Erfindung betrifft Hirudin-PA und dessen Derivate, ein Verfahren zu deren Herstellung, pharmazeutische Mittel, die diese Wirkstoffe enthalten und die Verwendung dieser Wirkstoffe.

Aus medizinischen Blutegeln (Hirudo medicinalis) sind schon einige Wirkstoffe isoliert worden, es handelt sich dabei um verschiedene Polypeptide, die als Proteinase-Inhibitoren wirken und teils Antithrombin-Wirkung haben. In der Literatur wird bezüglich dieser Wirkstoffe zwischen sogenannten Eglinen und Hirudinen unterschieden. In der DE—PS—28 08 396 sind z.B. zwei Egline beschrieben. Die Gewinnung von Roh-Hirudin ist z.B. in "die Pharmazie" *36*, 1981, S. 653—660 und in "Methods in Enzymology" Band 45, 1976, S. 669—678 beschrieben. Die vollständige Aminosäuresequenz der Hauptkomponente des Hirudins ist aus "FEBS 1104" (Federation of European Biochemical Societies, Vol. 165, 1984, S. 180—184) bekannt.

In FEBS Letters, 1983, 164 (2), 307—313 ist beschrieben, daß das C-terminale Ende des Hirudins dem enzymatischen Abbau durch endo- und exo-Peptidasen zugänglich ist. Es wurde gezeigt, daß die Abspaltung von 5-Aminosäuren vom C-terminalen Ende des Hirudins ein Produkt ergibt, dessen Fähigkeit die Amidase-Aktivität des Thrombins zu hemen, weitgehend verlorengegangen ist.

Oberraschenderweise wurde nun gefunden, daß Roh-Hirudin noch eine weitere Komponente enthält, die pharmakologisch wirksam ist.

Der Erfindung liegt daher die Aufgabe zugrunde, Blutegelextrakte auf neue pharmakologisch wirksame Substanzen zu untersuchen, insbesondere die Hirudinkomponente von Blutegelextrakten genauer auf neue Wirkstoffe zu untersuchen.

Die erfindungsgemäße Lösung dieser Aufgabe liegt in der Bereitstellung neuer Hirudinkomponenten, genannt Hirudin-PA, dessen Abbauprodukte und die desulfatierten Derivate davon.

Die Erfindung betrifft Hirudin-PA der Formel I:

```
                          10                  20
I T Y T D C T E S G Q N L C L C E G S N

                          30                  40
V C G K G N K C I L G S N G K D N Q C V

                          50                  60
T G E G T P K P Q S H N Q G D F E P I P

       *                       *
E D A Y D E                    Y=Tyrosin-O-Sulfat
```

dessen am N-Terminus um bis zu 2 Aminosäuren verkürzte Derivate, sowie die desulfatierten Derivate, bei denen in der obigen Formel I:

i) die Sulfatestergruppe am phenolischen Hydroxyl der Tyrosingruppe in Stellung 64 fehlt, oder

ii) die unter i) bezeichnete Sulfatestergruppe und die letzte oder die beiden letzten Aminosäuren, D, E (in Stellung 66 bzw. 66 + 65) fehlen,

und die pharmazeutisch brauchbaren Salze davon.

Eine bevorzugte Ausführungsform sind Hirudin-PA und dessen Derivate der Formel I, wobei die Aminosäurekette am N-Terminus um die Sequenz I oder IT verkürzt ist.

In den vorstehenden Formeln symbolisieren die angegebenen Buchstaben die der IUPAC-Nomenklatur entsprechenden proteinogenen Aminosäuren, die peptidisch verknüpft sind. Die Salze dieser Proteine sind ebenfalls Gegenstand dieser Erfindung.

Das erfindungsgemäße Hirudin-PA besteht aus insgesamt 66 Aminosäuren, sein Molekulargewicht beträgt 7087, seine spezifische Antithrombin-Aktivität ist 680—720 IU/mg und der Komplex mit Thrombin hat die Dissoziations-Konstante $K_i = 4 \times 10^{-11}$M.

Hirudin-PA und die Derivate davon sind dem bekannten Hirudin strukturell sehr ähnlich. An einigen wesentlichen Stellen der Proteinkette insbesondere am Anfang und Ende, unterscheiden sich jedoch die erfindungsgemäßen Produkte in charakterisischer Weise von der Sequenz des bekannten Hirudins. Daraus ergeben sich weitere Unterschiede, die von erheblicher praktischer Bedeutung sind. Die Tertiär-Struktur dieser Polypeptide wird durch drei Disulfid-Brücken stabilisiert. Auffallend ist beim Hirudin-PA die starksaure Sulfatmonoester-Gruppe am phenolischen Hydroxyl des Tyrosin-Restes in Stellung 64, die durch folgende

Teilstruktur-Formel wiedergegeben werden kann:

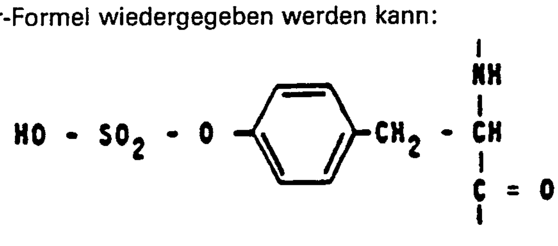

$[Tyr(SO_3H)^{64}]$

Diese Gruppierung liegt auch im bekannten Hirudin vor, dort allerdings in Stellung 63.

Bioligisch gehört Hirudin mit einem $K_i$-Wert von etwa $3 \times 10^{-11}$M zu den wirksamsten Thrombin-Inhibitoren, dabei wirkt es gegen Thrombin ganz spezifisch und inhibiert keine anderen Proteinasen der Blutgerinnungskaskade. Im Gegensatz zu Heparin üben die erfindungsgemäßen Hirudine ihren inhibierenden Einfluß auf Thrombin direkt aus. Von ihren pharmakologischen Wirkungen ist die Hemmung der Blutkoagulation wichtig. Die Wirkstoffe sind daher zur Prophylaxe von Thrombosen sehr geeignet. Unerwünschte Nebenwirkungen wurden bisher nicht festgestellt. Bei intravenöser Verabreichung an Hunde und Menschen war sogar bei ungewöhnlich hohen Dosen kein Einfluß auf Herzschlagfrequenz. Atmung, Blutdruck, Thrombozyten-Zahl, Fibrinogen und Hämoglobin zu beobachten. In Vergleichsversuchen waren die erfindungsgemäßen Verbindungen Heparin überlegen.

Zur Herstellung der erfindungsgemäßen Hirudine geht man am besten von einem der bekannten Blutegel-Extrakte aus. Wie oben bereits erwähnt, ist die Herstellung derartiger Extrakte beispielsweise in "Die Pharmazie" *36*, 1981, S. 653—660 oder in "Methods in Enzymology" Band 45, 1976, S. 669—678 beschrieben.

Man geht dabei so vor, daß man Blutegel (Hirudo medicinalis) oder die Kopfenden von Blutegeln zerkleinert, homogenisiert, den Gewebebrei mit wäßrigem Aceton oder Salz- bzw. Pufferlösung extrahiert, mit Äthanol einen wesentlichen Teil der Verunreinigungen des Extraktes fraktionierend ausfällt, die Lösung im Vakuum einengt, die eingeengte Lösung einer fraktionierenden Acetonfällung unterzieht, denn bei höherer Acetonkonzentration erhaltenen Niederschlag isoliert, mit Wasser extrahiert und den Extrakt lyophilisiert. Ausgehend von diesem an sich bekannten Lyophilisat ist das erfindungsgemäße Verfahren dadurch gekennzeichnet, daß man

A) das erhaltene Lyophilisat an einer Sephadex-Säule, die mit einem Puffer von pH 7,8 äquilibriert wurde, chromatographiert,

B) die Fraktionen mit Antithrombin-Aktivität lyophilisiert, das Lyophilisat entsalzt und erneut lyophilisiert,

C) mit dem Lyophilisat eine Anionenaustauschchromatographie an DEAE-Cellulose durchführt, wobei man mit einem Puffer von pH 6,5 äquilibriert und mit einem Puffer von pH 6,0 eluiert,

D) die Fraktionen mit Antithrombin-Aktivität lyophilisiert, entsalzt, lyophilisiert,

E) das Lyophilisat auf eine DEAE-Sephadex-Säule gibt, die mit einem Puffer von pH 6,0 äquilibriert wurde, mit einer Pufferlösung mit einem linearen pH-Gradienten, der aus einem Puffer von pH 5,0 und einem Puffer von pH 3,7 gebildet wird, eluiert,

F) die Fraktionen mit Antithrombin-Aktivität, die bei etwa pH 4,6 bis 4,7 eluiert werden lyophilisiert, entsalzt und wieder lyophilisiert,

G) schließlich das Lyophilisat an einer HPLC-Säule, die mit einem reversed phase-Trennmaterial vom Typ $C_{18}$ gefüllt ist, chromatographiert, wobei als Eluenten (A) 0,1% Trifluoroessigsäure in Wasser und (B) 0,1% Trifluoressigsäure in Acetonitril mit 40% (A) (v/v) dienen und isokratische Bedingungen (63% (A) + 37% (B)) herrschen und man vier Thrombin-hemmende Fraktionen erhält, von denen die erste Fraktion das gewünschte Hirudin-PA enthält;

H) zur Herstellung der desulfatierten Formen des Hirudin-PA die Sulfatmonoestergruppe am phenolischen Hydroxyl des Tyrosinrestes in Stellung 64, gewünschtenfalls auch die endständigen Aminosäuren D, E in Stellung 66, 65 einzeln oder gemeinsam hydrolytisch abspaltet, und

I) zur Hestellung der Abbauprodukte des Hirudin-PA dieses mit einer Peptidase inkubiert, die Produkte durch RP-HPLC auftrennt und die einzelnen Produkte isoliert und lyophilisiert.

Vorzugsweise führt man die Chromatographie in Stufe A an Sephadex G—75 durch, wobei man als Puffer von pH 7,8 eine wäßrige Lösung verwendet, die 50 mM Triäthanolamin und 300 mM oder 400 mM NaCl enthält. Sowohl dieser als auch den nachfolgend beschriebenen Pufferlösungen kann man zusätzlich z.B. 0,02 $NaN_3$ zusetzen.

Die Anionenaustrauschchromatographie führt man vorzugsweise an Whatman DE—52 oder DE—53 durch, wobei man als Äquilibrierungspuffer mit pH 6,5 vorzugsweise einen Ammoniumacetat-Puffer (30 mM $NH_4Ac$ und gegebenenfalls 0,02% $NaN_3$) und als Eluierungspuffer mit pH 6,0 vorzugsweise einen Natriumacetatpuffer der Zusammensetzung: 0,2 M NaAc und 0,19 M NaCl, verwendet. Als Eluierungspuffer kann man auch einen Salzgradienten verwenden, wobei man zunächst mit 0,03 M Ammoniumacetat, 0,19 M NaCl, pH 6,5 und anschließend mit 0,03 M Ammoniumacetat, 0,3 M NaCl, pH 65 arbeitet.

Bei der Sephadex DEAE—A25-Chromatographie verwendet man als Puffer von pH 6,0 vorzugsweise einen Natriumacetat-Puffer der zuvorbezeichneten Zusammensetzung. Für die Herstellung des pH-Gradienten verwendet man den gleichen Puffer, einerseits mit pH 5,0, andererseits mit pH 3,7. Diejenigen Fraktionen, die bei etwa pH 4,6 bis 4,7 eluiert werden, besitzen Antithrombin-Aktivität und werden als Hirudin Pool 1 bezeichnet. Fraktionen, die bei etwa pH 4,4 eluiert werden, werden als Hirudin Pool 2 isoliert. Der Antithrombin-Aktivität enthaltende Pool 1 wird dann lyophilisiert, entsalzt und erneut lyophilisiert. Die Entsalzungen werden in der Regel mit Sephadex G25 durchgeführt, wobei Wasser als Laufmittel dient. Für die HPCL-Trennung kann man z.B. eine Supelco-Säule Typ LC—18—DB, 5 µm oder eine Lichrospher-Säule Typ 100 CH—18/2, 5 µm 4,6 × 250 ml von Merck verwenden.

Man arbeitet einer derartigen Säule beieinem Fluß von 1 ml/Minute, bei einer Temperatur von 25°C und detektiert bei 214 und 254 nm.

Bei dieser HPLC-Trennung des Hirudin Pool 1 erhält man vier Thrombin-hemmende Fraktionen. Der

mengenmäßig größe Teil ist mit dem aus der Literatur schon bekannten Hirudin identisch. Eine andere kleine Fraktion unterscheidet sich vom bekannten Hirudin nur durch den Austausch einer Aminosäure. Das erfindungsgemäße Hirudin-PA ist in der ersten und zweitstärksten Fraktion enthalten. Es ist ein vollkommen neuer Inhibitor-Typ mit N-terminalem Ile. Es wurde wie folgt charakterisiert und sequenziert:

Das Protein der vorstehend erwähnten Hirudin-PA-Fraktion wurde an einer Festphase immobilisiert und in diesem Zustand dem sogenannten Edman-Abbau unterworfen. Die hierbei angewandte Standardtechnik der automatischen Festphasensequenzierung ist z.B. in zwei Obersichtsartikeln von W. Machleidt "Modern Methods in Protein-Chemistry-Revue Articles", erschienen 1983 bei Walter de Gruyter & Co.-Berlin, New York, und von Richard A. Laursen und W. Machleidt in "Methods of of Biochemical Analysis", Vol. 26, S. 201—284, 1980, beschrieben. Hierbei wird das zu sequenzierende Peptid über funktionelle Gruppen der Aminosäure-Seitenketten kovalent an den unlöslichen Träger, die Festphase, gebunden. An der Festphase durchläuft es die zyklischen Reaktionen des Edman-Abbaus, die zur Abspaltung der jeweiligen N-terminalen Aminosäuren führen. Die Bindung des Peptids an die Festphase muß unter den Bedingungen des Edman-Abbaus stabil sein, sie darf jedoch den Abbau bis zum C-Terminus nicht behindern.

Bei dieser Arbeitsweise ist das Phenylthioharnstoff-Derivat der Cysteinsäure zur positiven Identifizierung der Position der Cysteinreste hervorragend geeignet. Zur Klärung de N-terminalen Sequenz wurden daher Sequenzläufe mit nativem und oxidiertem Hirudin-PA durchgeführt. Je Lauf wurden 25 nMol Protein eingesetzt, die zur Hälfte über Aminogruppen an Diisothiocyanatglas (DITC-Glas) und zur anderen Hälfte durch Carbodiimid-Kopplung der Carboxyl-Gruppen an Aminopropylglas (APG) immobilisiert waren. Diese beiden Immobilisierungstechniken sind z.B. auf den Seiten 270 und 273 der oben erwähnten Literaturstelle W. Machleidt "Modern Methods in Protein-Chemistry, 1983" beschrieben. Da Lysinrese in hoher Ausbeute über ihre α-Aminogruppe an DITC-Glas koppeln, hat die Mischkopplung den Vorteil, daß in einem Sequenzlauf sowolhl die N-terminale Aminosäure wie auch die Positionen der Lysinreste erfaßt werden können.

Es gelang, die sequenz des Hirudin-PA zunächst bis Position 46 aufzuklären (Abb. 1). Die Positionen der Cysteinreste und Lysinreste wurden positiv nachgewiesen, jedoch wurden ihre Phenylthioharnstoff (PTH)-Derivate nicht quantifiziert. Offene Kreise symbolisieren die PTH-Derivate von Asp, Glu, Ser, Thr und Pro. Sie repräsentieren solche Aminosäuren, deren Ausbeute an PTH-Derivaten niedriger ausfiel. Die repetitive Ausbeute des Sequenzlaufes von oxidiertem Hirudin-PA (Abb. 1) wurde für die Schritte 10—34 zu 94% berechnet. Ann Position 35, dem Lys 35-Ankerpukt, fällt die Ausbeute an PTH-Aminosäuren sprunghaft ab. Diese Daten deuten an, daß über den letzten Aminogruppen-Ankerpunkt Lys 47 nur noch wenig Material gebunden war und gleichzeitig die Ausbeuten für die Carboxylgruppen-Kopplung niedrig lagen.

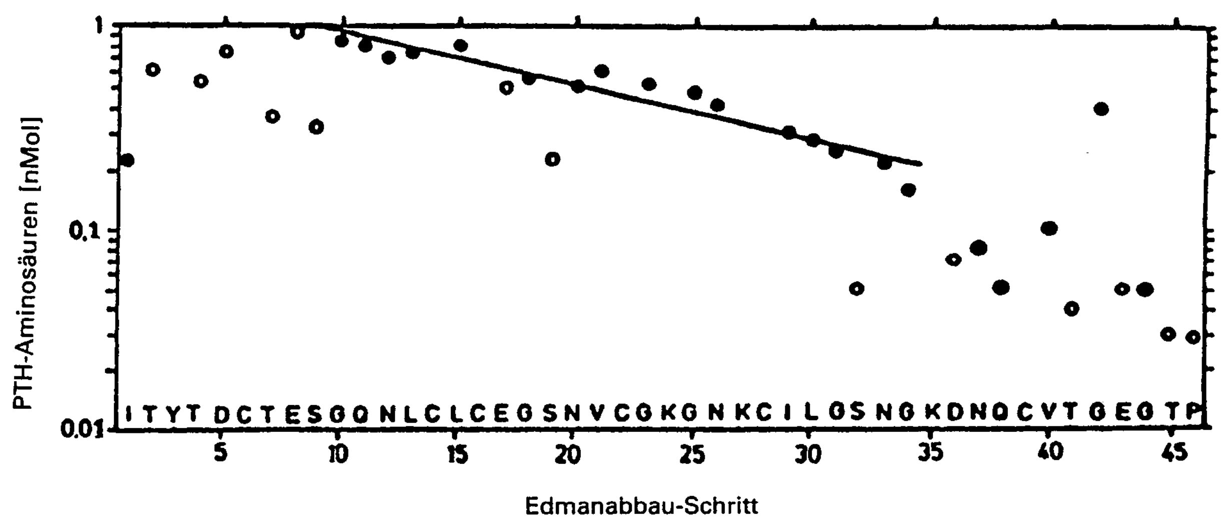


*Abb. 1.*

Ausbeuten an PTH-Aminosäuren des oxidierten Hirudin PA

Zur Berechnung der Regressionsgeraden wurden die ausgefüllten Kreise der Positionen 10—34 herangezogen. Aus der Steigung ergab sich eine repetitive Ausbeute von 94%. Offene Kreise wurden für Asp, Glu, Pro, Thr und Ser verwendet, die entweder teilweise über Seitenketten am Träger fixiert sind (Asp, Glu), oder unvollständig gespalten werden (Pro) oder während des Abbaus teilweise zestört werden (Thr, Ser). Lysin wurde zwar positiv identifiziert, jedoch nicht quantifiziert.

Tryptische Spaltung von oxidiertem Hirudin PA

Es wurden 19 Proteingipfel bei der Auftrennung eines tryptischen Spaltansatzes mittels RP—HPLC erhalten. Die Charakterisierung der Peptide erfolge über Aminosäureanalyse und N-terminale Bestimmung

mit einem ein-Schritt-Handedmanabbau (DABITC/PITC doppelkupplungsmethode*). Das Peptid TR 3 wurde als Tripeptid G—N—K identifiziert, welches den bekannten Positionen 25—27 zugeordnet wurde. Das Peptid TR 16 beinhaltete das mit der bisherigen Sequenzinformation überlappende C-terminale Peptid, beginnend mit Position 36 (Asp). Dieses wurde nach Ankoppeln an APG dem automatischen Festphasenedmanabbau unterworfen. Durch Seitenkettenfixierung von Asp und Glu bedingt lagen die Ausbeuten dieser PTH-Derivate wiederum signifikant niedriger. Für die anderen Derivate gilt das vorher Gesagte. Die Ausbeute des zweiten Edmanabbauschrittes (Asn) wurde als 100% angesetzt und die übrigen Ausbeuten darauf bezogen. Aus der Steigung der Regressionsgeraden für die ausgefüllten Kreise ergab sich eine repetitive Ausbeute von 93,3% (Abb. 2).

Ein Vergleich der Aminosäureanalyse und der Aminosäurezusammensetzung nach Sequenzierung ergab eine Diskrepanz von einer Aminosäure: innerhalb der Sequenz war ein Glx zuwenig bestimmt worden. Diese Aminosäure musste den C-Terminus bilden, da sonst keine Hinweise auf ein zusätzliches Glx bemerkt worden waren. Zur Klärung der C-terminalen Sequenz und der Frage, ob der Tyrosinrest ebenso wie im Hirudin ein Tyrosin-O-Sulfatrest sei, sollte eine C-terminale Sequenzierung mit Carboxypeptidase Y erfolgen.

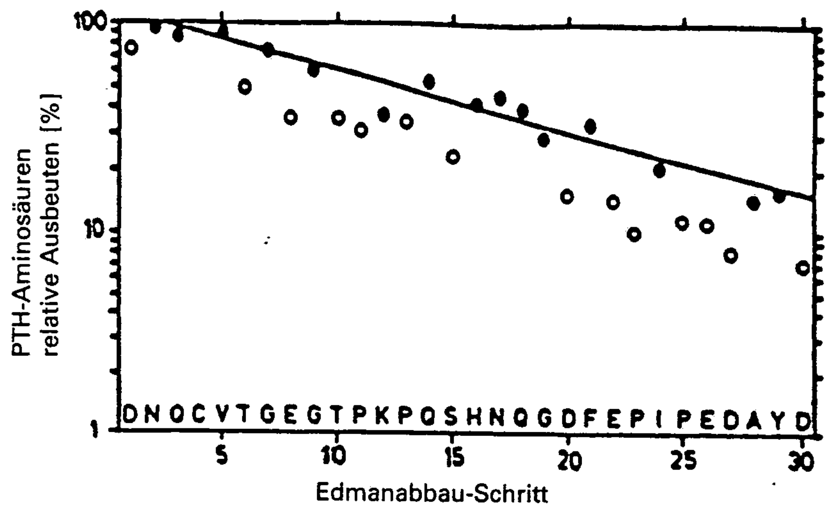


*Abb. 2:*
Ausbeuten an PTH-Aminosäuren des tryptischen Peptides TR 16

Die Ausbeute des 2. Abbauschrittes nach Edman wurden als 100% gesetzt und die übrigen Ausbeuten darauf bezogen. Zur Berechnung der Regressionsgraden wurden die ausgefüllten Kreise herangezogen, aus deren Steigung eine repetitive Ausbeute von 93,3% erhalten wurde. Offene Kreise wurden für die Aminosäuren Asp, Glu, Pro, Thr und Ser verwendet (s. Abb. 1).

C-terminale Sequenzierung des Hirudin PA mit Carboxypeptidase Y (CPY)

Die C-terminale Sequenzierung von Hirudin PA erfolgte von nativem Hirudin Pa, dem Peptid TR 16 und dem Peptid TGR 16, welches zuvor 30 Minuten bei 60°C mit 50%iger TFA inkubiert worden war. Es wurde zunächst eine rasche Abspaltung von Glutaminsäure beobachtet, ehe es zu einer Freisetzung von Asparaginsäure und weiteren Aminosäuren kam (Abb. 3) Die Freisetzung von Tyrosin wurde nur in dem TFA-behandeltem Peptid TR 16 verfolgt, während die Einwirkung von CPY auf die beiden anderen Proben zur Abspaltung einer Aminosäure führte, die sich wie synthetisches Tyrosin-O-Sulfat verhielt, Damit war der Tyrosinrest in Position 64 des Hirudin PA ebenfalls als Tyrosin-O-Sulfatrest identifiziert. Das überraschendste Ergebnis war jedoch die Bestimmung von Glu als C-terminaler Aminosäure. Da während der automatischen Sequenzierung des TR 16 kein Anhaltspunkt für diesen Rest erhalten worden war, muss er vollständig über C-terminale und Seitenkettencarboxylgruppen an den Träger gekoppelt worden sein, womit er einer Identifizierung nicht zugänglich war. Asp wird durch CPY nur schlecht hydrolysiert und bildet somit einen limitierenden Faktor im Abbau. Eine Zuordnung der Reihenfolge der C-terminalen Aminosäuren ist dadurch erschwert, zumal zwei Asp nahe benachbart in der Sequenz vorkommen und Pro nicht detektiert wurde. Die C-terminale Sequence durch CPY-Abbau soll daher nur zu -Tyr-Asp-Glu angegeben werden.

---

* Chang, Brauer + Wittman-Liebold, 1978, FEBS-Lett. *93*, S. 205—214.

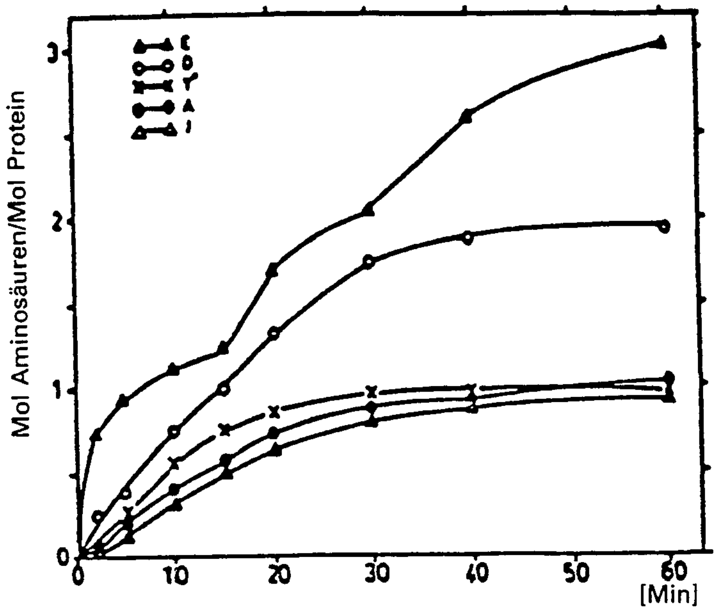


*Abb. 3:*
Zeitlicher Verlauf der CPY-katalysierten Freisetzung der Aminosäuren vom C-Terminus des Hirudin PA

Hirudin PA wurde mit CPY (40/1 = w/w) in einem 10 mM Phosphatpuffer pH 4,7 bei 37°C inkubiert. Von dem Inkubationsansatz wurden zu bestimmten Zeiten aliquote Proben entnommen, auf pH 2,0 eingestellt und lyophilisiert. Das Lyophilisat wurde am Aminosäureanalysator auf freigesetzte Aminosäuren analysiert. Prolin wurde nicht detektiert. Die C-terminale Sequenz wurde zu -Tyr-Asp-Glu bestimmt.

Die gesamte Sequenz des Hirudin-PA ist in Abb. 4 dargestellt.

10 20
I T Y T D C T E S G Q N L C L C E G S N

30 40
V C G K G N K C I L G S N G K D N Q C V

50 60
T G E G T P K P Q S H N Q G D F E P I P

E D A Y* D E        Y*=Tyrosin-O-Sulfat

*Abb. 4:*
Sequenz der Aminosäurebausteine im Hirudin PA, identifiziert mittels:

⟵⟶ direktem Edmanabbau von nativem und oxidierten Hirudin PA

← — → Edmanabbau der tryptischen Peptide

← ← ← enzymatischem Abbau durch CPY

Die erfindungsgemäßen Abbauprodukte des Hirudin-PA sind durch enzymatischen Abbau des Hirudin-PA in Form einer limitierten Proteolyse erhältlich. Dazu verwendet man Peptidasen, wie Carboxypeptidasen, d.h. Proteasen, die eine Aminosäurekette vom Carboxylende her spalten und/oder Aminopeptidasen, d.h. Proteasen, die eine Aminosäurekette vom Aminoende her angreift. Geeignete Proteasen, die auch am Träger gebunden sein können, sind Carboxypeptidasen A, Leucinaminopeptidase und die Kathepsine A, B, C un D; insbesondere sind jedoch Carboxypeptidase Y und Kathepsin C geeignet.

Es ist bekannt, daß Kathepsin C als Dipeptidylaminopeptidase sequentiell Dipeptide vom unsubstituierten Aminoende vom Protein abspaltet. Es wurde jedoch gefunden, daß Kathepsin C bei der Proteolyse von Hirudin auch C-terminale Exopeptidase-Aktivität besitzt, so daß sich der Abbau von Hirudin-PA mit Kathepsin C auch vom C-terminalen Ende her bewerkstelligen läßt.

Vorzugsweise erfolgt die Proteolyse von Hirudin-PA mit Kathepsin C. Die Inkubation führt man

zweckmäßigerweise bei 37°C durch. Das pH-Optimum de enzymatischen Aktivität von Kathepsin C liegt im schwach sauren Milieu bei pH 5 bis 6.

Die Auftrennung des nach der Proteolyse erhaltenen Produktgemisches erfolgt mittels HPLC. Als Säule kann man beispielweise eine Supelco-Säule LC—18—DB oder eine Lichrospher-Säule 100 CH—18/2 verwenden. Als Eluierungsmittel hat sich ein Gradient aus 0,1% Trifluoressigsäure in Wasser (V/V; Puffer A) und 0,1% Trifluoroessigsäure in Acetonitril mit 40% Puffer A (V/V) als zweckmäßig erwiesen.

Erfindungsgemäß sind die Desulfatohirudine-PA erhältlich, indem man im Hirudin-PA der oben angegebenen Formel die als Schwefelsäure-monoester vorhandene phenolische Hydroxygruppe des Tyrosin-Restes in Stellung 64 freisetzt.

Die Freisetzung dieser Gruppe entsprechend dem Schema

$$HO—SO_2—OP—Pept \rightarrow HO—Pept$$

(worin Pept den restlichen Teil von Hirudin darstellt) kann z.B. durch Hydrolyse erfolgen, wobei sowohl chemische als auch biologische Methoden zu diesem Zweck anwendbar sind.

Bei einer chemischen Freisetzung wird vorzugsweise unter den allgemeinen Bedingungen der säurekatalysierten Hydrolyse gearbeitet, wie unter der Einwirkung einer verdünnten, z.B. einer etwa 2- bis etwa 4N wäßrigen Salzsäurelösung, vorteilhaft in Trifluoressigsäure, als Reaktionsmedium oder mit wasserhaltiger Trifluoressigsäure allein als Reaktions- und Lösungsmittel. Um die Gefahr der hydrolytischen Spaltung von Peptidbindungen auf einem Minimum zu halten, ist es empfehlenswert, unter milden Reaktionsbedingungen, z.B. bei Temperaturen, welche Zimmertemperatur nicht übersteigen, zu arbeiten und den Fortschritt der Hydrolyse analytisch, z.B. durch Dünnschichtchromatographie, zu verfolgen.

Vornehmlich erfolgt jedoch die Hydrolyse auf biologischem Wege, insbesondere durch die Anwendung von spezifischen Ensymen, Arylsulfatasen, welche die phenolischen Sulfatestergruppen zu freien phenolischen Gruppen unter milden Bedingungen spalten. Die biologische Freisetzung der sulfatierten Hydroxylgruppe kann mit Hilfe eines geeigneten Enzympräparats mit angereichertem Wirkstoff oder eines isolierten Enzyms erfolgen, oder aber man kann ein geeignetes Enzymsystem in situ einsetzen, d.h. wie es in einem lebenden oder abgetöten biologischen Material, z.B. einem wachsenden oder ruhenden Mikroorganismus, einer Zellkultur, einem Zellhomogenat oder einem Autolysat, unmittelbar vorliegt. Einer der größten Vorteile der biologischen Hydrolyse ist ihre hohe Selektivität, die nur die gewünschte Spaltung der Monosulfatester-Bindung bewirkt, ohne die übrigen funktionellen Gruppen, vor allem die peptidischen Bindungen, im empfindlichen Ausgangsstoff anzugreifen. Vornehmlich werden die erfindungsgemäßen Verbindungen hergestellt, indem man Hirudin-PA in einer wäßrigen, vorzugsweise gepufferten Lösung oder Suspension mit einem individuellen Arylsulfatase-Präparat, z.B. der Arylsulfatase aus Helix pomatia, bei einer für enzymatische Prozesse gebräuchlichen Temperatur, wie etwa 20 bis 45 und vorzugsweise 25 bis 30°C, behandelt. Vorzugsweise wird bei einer schwach sauren Reaktion, d.h. bei einem pH von etwa 4 bis etwa 7, insbesondere von etwa 5 bis etwa 6, gearbeitet, welcher mit einem Puffer, wie einer etwa 0,03- bis etwa 0,3-molaren Lösung eines Salzes einer organischen Carbonsäure mit einem Alkalimetall oder mit einer organischen Base, z.B. mit Natriumacetat at bzw. insbesondere Pyridinacetat (vom pH etwa 5,4), eingestellt wird. Das Verhältnis des eingesetzten Enzyms zum Substrat (Hirudin) richtet sich im allgemeinen nach der Aktivität des jeweiligen Präparats und beträgt üblicherweise von etwa 1:2 bis etwa 1:100, insbesondere von etwa 1:5 bis etwa 1:20; es ist vorteilhaft, Enzympräparate von höchstmöglichem Reinheitsgrad und Aktivität zu verwenden. Da die Arylsulfatasen nicht nur die Abspaltung, sondern auch die Einführung der Sulfatgruppe katalysieren und das Eiknstellen eines Gleichgewichts der Ausgangs- und Endstoffe bewirken, ist es vorteilhaft, für jedes Enzympräparat durch Vorversuche die optimale Konzentration, das Mengenverhältnis zum Substrat und den Zeitverlauf der Desulfatisierung festzustellen. In der Regel ist aber die Reaktion schon nach wenigen Minuten beendeet; die Qualität des Reaktionsproduktes wird auch bei längerem (bis etwa 4 stündigen) Kontakt mit aktivem Enzym (z.B. beim Stehen des Reaktionsgemisches) nicht beeinträchtigt.

Der Verlauf der enzymatischen Desulfatisierung kann bioanalytisch an entnommenen Proben verfolgt werden: praktisch geht man z.B. so vor, daß man durch kurzes (etwa 3 minütiges) Erhitzen der Probe auf etwa 100°C die Enzymaktivität zerstört und das Substrat mit einer Carboxypeptidase Y behandelt. (Die Carboxypeptidase Y baut die Peptidkette von der Carboxyseite her ab, indem die Aminosäuren nacheinander durch Spalten der jeweiligen Amidbindungen abgespalten werden.) Üblicherweise ist der Abbau der Peptidkette nach etwa 15 min so forgeschritten, daß die betreffende, sulfatisierte und/oder freie 64-ständige Aminosäure ($Tyr^{64}$) vollständig abgespalten und somit der Bestimmung in einem konventionellen Aminosäure-Analysator zugänglich ist.

Die E,D-verkürzten Desulfatohirudine entstehen durch Abspaltung der beiden C-terminalen Aminosäurebausteine Glu und Asp im Laufe der Hydrolyse von Hirudin-PA. Die Auftrennung des dabei anfallenden Gemisches kann beispielsweise durch präparative HPLC-Chromatographie erfolgen. Die Desulfatohirudine-PA besitzen die gleichen biologischen Eigenschaften wie das Hirudin-PA.

Die erfindugnsgemäßen Verbindungen können in freier Form und als Salz vorliegen. Da sie freie Aminogruppen bzw. Amidinogruppen enthalten, können sie auch als Säureadditionssalze vorliegen. Als Säureadditionssalze kommen insbesondere physiologisch verträgliche Salze mit üblichen, therapeutische

anwendbaren Säuren in Betracht. Als anorganische Säuren sind die Halogenwasserstoffsäuren, z.B. Chlorwasserstoffsäure, aber auch Schwefelsäure und Phosphor-bzw. Pyrophosphorsäure zu nennen. Als organische Säuren kann man z.B. Sulfonsäuren, z.B. Dibenzol- oder p-Toluolsulfonsäure oder Niederalkansulfonsäuren, wie Methansulfonsäure, andererseits Carbonsäure, wie Essigsäure, Milchsäure, Palmitin- und Stearinsäure, Äpfelsäure, Weinsäure, Ascorbinsäure und/oder Citronensäure verwenden. Da die erfindungsgemäßen Verbindungen andererseits auch freie Carboxylgruppen enthalten, können sie auch als Salz einer Base vorliegen, z.B. als Natrium-, Kalium-, Kalzium- oder Magnesiumsalz oder als Amoniumsalz oder als Salz einer physiologisch verträglichen organischen stickstoffhaltigen Base.

Je nach Arbeitsweise lassen sich somit die erfindungsgemäßen Verbindungen in freier Form oder in Form von Säureadditionssalzen, inneren Salzen oder Salzen mit Basen gewinnen. Aus den Säureadditionssalzen kann in an sich bekannter Weise die freie Verbindung gewonnen werden. Von letzterer wiederum lassen sich durch Umsetzen mit Säuren, z.B. mit solchen die die oben genannten Salze bilden, und Eindampfen oder Lyophilisieren therapeutisch anwendbare Säureadditionssalze gewinnen. Die inneren Salze können durch Einstellen des pH auf einen geeigneten Neutralpunkt gewonnen werden.

Die Erfindung betrifft auch pharmazeutische Zubereitungen, welche die erfindungsgemäßen Verbindungen oder deren pharmazeutisch verwendbare Salze, gegebenenfalls zusammen mit einem pharmazeutischen Träger und/oder Hilfsstoffen enthalten.

Diese Zusammensetzungen können insbesondere bei den oben angegebenen Indikationen Verwendung finden, wenn sie z.B. parenteral (wie intravenös, intracutan, intramuskulär oder subcutan), oral oder topisch verabreicht werden. Die Dosierung hängt in erster Linie von der spezifischen Verabreichungsform und vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden; die dazu erforderlichen Methoden zur Bestimmung von relevanten Blutfaktoren sind dem Fachmann geläufig. Im Normalfall liegt bei einer Injektion die therapeutisch wirksame Menge der erfindungsgemäßen Verbindungen im Dosisbereich von etwa 0,005 bis etwa 0,1 mg/kg Körpergewicht. Bevorzugt wird der Bereich von etwa 0,01 bis etwa 0,05 mg/kg Körpergewicht. Die Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg der erfindungsgemäßen Verbindung. Neben dem Wirkstoff enthalten diese pharmazeutischen Zusammensetzungen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2 bis 0,3% 4-Hydroxybenzoesäure-methylester oder -ethylester, enthalten können.

Ein Präparat für die topische Anwendung kann als wäßrige Lösung, Lotion oder Gelee, ölige Lösung oder Suspension, oder fetthaltige oder insbesondere Emulsions-Salbe vorliegen. Ein Präparat in Form einer wäßrigen Lösung erhält man beispielsweise dadurch, daß man den erfindungsgemäßen Wirkstoff oder ein therapeutisch anwendbares Salz davon in einer wäßrigen Pufferlösung von pH 4 bis 6,5 löst und gewünschtenfalls einen weiteren Wirkstoff, z.B. ein Antiinflammatorikum, und/oder ein polymeres Haftmittel, z.B. Polyvinylpyrrolidon, und/oder ein Konservierungsmittel zufügt. Die Konzentration des Wirkstoffs beträgt etwa 0,08 bis etwa 1,5 mg, vorzugsweise 0,25 bis 1,0 mg, in etwa 10 ml einer Lösung bzw. 10 g eines Gelees.

Eine ölige Applikationsform für die topische Verabreichung erhält man beispielsweise durch Suspendieren des erfindungsgemäßen Wirkstoffs oder eines therapeutisch anwendbaren Salzes davon in einem Öl, gegebenenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z.B. Glycerinmonostearat, Sorbitanmonolaurat, Sorbitanmonostearat oder Sorbitanmonooleat. Eine fetthaltige Salbe erhält man z.B. durch Suspendieren des erfindungsgemäßen Wirkstoffs oder der Salze in einer streichbaren Fettgrundlage, gegebenenfalls unter Zusatz eines Tensids vom HLB-Wert unter 10. Eine Emulsionssalbe erhält man durch Verreiben einer wäßrigen Lösung des erfindungsgemäßen Wirkstoffs oder der Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0,08 bis etwa 1,5 mg, vorzugsweise 0,25 bis 1,0 mg, in etwa 10 g der Grundmasse.

Neben den oben beschriebenen und ihnen analogen pharmazeutischen Zusammensetzungen, welche für einen direkten medizinischen Einsatz am Körper des Menschen oder eines Säugetieres bestimmt sind, betrifft die vorliegende Erfindung auch pharmazeutische Zusammensetzungen und Präparate zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. Dialyse in künstlichen Nieren), Konservierung oder Modifizierung (z.B. Haemoseparation) unterzogen wird. In ihrer Zusammensetzung sind derartige Präparate, wie Vorratslösungen oder auch Zubereitungen in Einzeldosis-form, den oben beschriebenen Injektionspräparaten ähnlich; zweckmäßigerweise wird aber die Wirkstoffmenge bzw. -konzentration auf das Volumen des zu behandelnden Blutes oder, noch exakter, auf dessen Thrombingehalt bezogen. Dabei ist zu beachten, daß die erfindungsgemäßen Wirkstoffe (in freier Form)

(a) eine etwa 5fache Gewichtsmenge Thrombin völlig desaktivieren;

(b) auch in größeren Mengen physiologisch harmlos sind; und

(c) vom zirkulierenden Blut auch in hohen Konzentrationen sehr schnell ausgeschieden werden, so daß keine Gefahr einer Überdosierung, auch z.B. bei Transfusionen, besteht. Je nach dem spezifischen Zweck beträgt die geeignete Dosis etwa 0,01 bis etwa 1,0 mg Wirkstoff/l Blut, wobei die obere Grenze ohne Gefahr noch weit überschritten werden darf.

Zum Gegenstand der vorliegenden Erfindung gehört auch die bioanalytische Anwendung der erfindungsgemäßen Verbindungen und ihrer Salze zur Bestimmung von Thrombin, sowie die diesem Zweck dienenden, die erfindungsgemäßen Wirkstoffe enthaltenden Präparate, z.B. Feststoffgemische und vor allem Lösungen, insbesondere wäßrige Lösungen; diese können neben einer genauen Menge bzw. Konzentration der erfindungsgemäßen Wirkstoffe (auch in Form eines Salzes) zweckmäßig noch inerte Hilfsstoffe enthalten, z.B. die oben bei Injektionspräparaten erwähnten, welche beispielsweise eine stabilisierende und/oder konservierende Aufgabe haben. Diese Präparate werden bei Bioanalysen in analoger, bekannter Weise beispielsweise zur Bestimmung von Thrombin verwendet.

Darüber hinaus sind die erfindungsgemäßen Verbindungen zur Blutkonservierung brauchbar. Zu diesem Zweck setzt man den erfindungsgemäßen Verbindungen Blutkonserven vorteilhaft in einer Menge von 0,1—2 Gew.-% zu.

In der vorliegenden Beschreibung und in den Ansprüchen werden die Kurzbezeichnungen für Aminosäuren und ihre Reste im Einklang mit den allgemein angenommenen nomenklatorischen Regeln angewendet und beziehen sich auf α-Aminosäuren und deren Reste der in der Natur vorkommenden L-Reihe.

### Beispiel 1

2,5 g Rohhirudin (gewonnen durch fraktionierte Fällung mit Aceton) werden in 20 ml Elutionspuffer gelöst und auf eine Sephadex G—75 medium-Säule (3,8 × 150 cm) aufgetragen. Als Elutionspuffer verwendet man 0,05 M Triethanolamin, 0,4 M NaCl, 0,02% $NaN_3$, pH 7,8. Durchfluß 55 ml/h; Fraktionsvolumen: 9,7 ml.

Die Fraktionen mit Inhibitor-Aktivität gegenüber Thrombin wurden vereinigt (300 bis 400 ml), in einer Amicon Ultrafiltrationszelle mit UM 05-Membran entsalzt und anschließend lyophilisiert.

Das Lyophilisat wurde einer Anionenaustauschchromatographie an DEAE-Cellulose (DE—52, Whatman; 2,5 × 100 cm) unterzogen. Äquilibierungspuffer: 0,03 M Ammoniumacetat, pH 6,5. Durchfluß: 25 ml/h; Fraktionsvolumen: 8,3 ml.

Es wurden 1,4 g des Inhibitors nach Lösen in 10 ml des Äquilibrierungspuffers und Nachstellen des pH-Wertes aufgetragen.

Die Säule wurde so lange mit Äquilibrierungspuffer entwickelt, bis die chymotryptische Hemmaktivität eluiert worden war. Die Eluierung erfolgte mit einem Natriumacetatpuffer vom pH 6,0 mit der Zusammensetzung: 0,2 M NaAc, 0,19 M NaCl, 0,02% $NaN_3$.

Die thrombinhemmenden Fraktionen wurden vereinigt, mittels Ultrafiltration wie oben entsalzt und lyophilisiert.

Für die anschließende Chromatographie an DEAE-Sephadex A—25 (Säule 1,9 × 68 cm) verwendete man einen Äquilibrierungspuffer aus 0,2 M Natriumacetat, 0,19 M NaCl, pH 6,0. Durchfluß: 11 ml/H; Fraktionsvolumen: 3,7 ml.

Es wurden 63 mg des Inhibitors nach Lösen in 10 ml des Äquilibrierungspuffers und Nachstellen des pH-Wertes aufgetragen.

Die Säule wurde 2 Stunden mit Äquilibrierungspuffer entwickelt, anschließend auf den gleichen Puffer vom pH 5,0 umgestellt und weitere 14 Stunden äquilibriert.

Die Säule wurde mit einem linearen pH-Gradienten eluiert (gleicher Puffer wie oben, pH 3,7).

Die thrombinhemmende Fraktion, die bei etwa pH 4,6 bis 4,7 eluiert wurde, wurde auf ca. 3 ml konzentriert.

Anschließend wurde lyophilisiert, an Sephadex G—25 unter Verwendung von Wasser als Laufmittel entsalzt und erneut lyophilisiert.

Das Lyophilisat wurde mittels RP—HPLC an Lichrospher 100 CH—18/2, 5 μm, 4,6 × 250 mm aufgetrennt. Eluiert wurde mit 0,1% Trifluoressigsäure in Wasser (V—V; Puffer A) und 0,1% Trifluoressigsäure mit Acetonitril mit 40% Puffer A (V—V; Puffer B). Die Elution erfolgte isokratisch mit einer Lösung aus 63% Puffer A und 37% Puffer B (V—V). Fluß: 1 ml/Minute. Detektion: bei 214 mm und 254 nm.

Das bei einer Retentionszeit von 9,6 Minuten erhaltene Eluat enthält Hirudin PA, das nach Lyophilisierung in Substanz erhalten wurde.

### Beispiel 2

Herstellung der desulfatierten Derivate durch Inkubation mit Arylsulfatase (ARS)

ARS-Stammlösung: 0,1 ml ARS-Suspension wurde mit 0,1 M Ammoniumacetat, pH 5,5 auf 2,5 ml verdünnt und an einer PD 10-Säule entsalzt.

Hirudin-PA Stammlösung: 2 mg/ml Puffer.

Puffer: 0,1 M Ammoniumacetat, pH 5,5 Hirudin-PA-Stammlösung und ARS-Stammlösung wurden im Verhältnis von 1:10 (V/V) gemischt und bei 25°C inkubiert. Der zeitliche Verlauf der Reaktion wurde mittels

HPLC verfolgt. Nach 24 Stunden Inkubationszeit wurde 3 Minuten auf 90°C erhitzt und das Gemisch mittels HPLC unter folgenden Bedingungen aufgetrennt:

Säule: Lichrospher 100 CH—18/2, 5 µm, 4,6 × 250 mm.
Puffer A: 0,1% Trifluoressigsäure in Wasser (V/V)
Puffer B: 0,1% Trifluoressigsäure in Acetonitril mit 30% Puffer A (V/V)
Fluß: 1 ml/Minute
Detektion: bei 214 nm
Temperatur: 25°C
Elution: isokratisch mit 66% Puffer A und 34% Puffer B (V/V)

Beispiel 3

Herstellung der Abbauprodukte des Hirudin-PA

Hirudin-PA Lösung (2 mg/ml Puffer) Katepsin C-Stammlösung (20 U/1,14 ml) werden im Verhältnis von 1:5 (V/V) gemischt und bei 37°C inkubiert. Der Verlauf der Proteolyse wird mittels RP—HPLC verfolgt. Der Spaltansatz wird nach 5-stündiger Inkubation präparativ mittels HPLC aufgretrennt. Die Bedingungen waren wie im Beispiel 2 angegeben.

**Patentansprüche**

1. Hirudin-PA der Formel I:

```
                         10                   20
I T Y T D C T E S G Q N L C L C E G S N

                         30                   40
V C G K G N K C I L G S N G K D N Q C V

                         50                   60
T G E G T P K P Q S H N Q G D F E P I P

       *                        *
E D A Y D E                     Y=Tyrosin-O-Sulfat
```

in der die angegebenen Buchstaben die der IUPAC-Nomenklatur entsprechenden proteinogenen Aminosäuren symbolisieren, die peptidisch verknüpft sind, und dessen am N-Terminus um bis zu 2 Aminosäuren verkürzte Derivate, sowie die desulfatierten Derivate, bei denen in der obigen Formel I:

i) die Sulfatestergruppe am phenolischen Hydroxyl der Tyrosingruppe in Stellung 64 fehlt, oder

ii) die unter i) bezeichnete Sulfatestergruppe und die letzte oder die beiden letzten Aminosäuren D, E (in Stellung 66 bzw. 66 + 65) fehlen,

und die pharmazeutisch brauchbaren Salze davon.

2. Verfahren zur Herstellung von Hirudin-PA und dessen Derivaten nach Anspruch 1, wobei man Blutegel (Hirudo medicinalis) oder die Kopfenden von Blutegeln zerkleinert, homogenisiert, den Gewebebrei mit wäßrigem Aceton oder Salz- bzw. Pufferlösung extrahiert, mit Äthanol einen wesentlichen Teil der Verunreinigungen des Extraktes fraktionierend ausfällt, die Lösung im Vakuum einengt, die eingeengte Lösung einer fraktionierten Acetonfällung unterzieht, den bei höherer Acetonkonzentration erhaltenen Niederschlag isoliert, mit Wasser extrahiert und den Extrakt lyophilisiert, dadurch gekennzeichnet, daß man

A) das erhaltene Lyophilisat an einer Sephadex-Säule, die mit einem Puffer von pH 7,8 äquilibriert wurde, chromatographiert,

B) die Fraktionen mit Antithrombin-Aktivität lyophilisiert, das Lyophilisat entsalzt und erneut lyophilisiert,

C) mit dem Lyophilisat eine Anionenaustauschchromatographie an DEAE-Cellulose durchführt, wobei man mit einem Puffer von pH 6,5 äquilibriert und mit einem Puffer von pH 6,0 eluiert,

D) die Fraktionen mit Antithrombin-Aktivität lyophilisiert, entsalzt, lyophilisiert,

E) das Lyophilisat auf eine DEAE-Sephadex-Säule gibt, die mit einem Puffer von pH 6,0 äquilibriert wurde, mit einer Pufferlösung mit einem linearen pH-Gradienten, der aus einem Puffer von pH 5,0 und einem Puffer von pH 3,7 gebildet wird, eluiert,

F) die Fraktionen mit Antithrombin-Aktivität, die bei etwa pH 4,6 bis 4,7 eluiert werden lyophilisiert, entsalzt und wieder lyophilisiert,

G) schließlich das Lyophilisat an einer HPLC-Säule, die mit einem reversed phase-Trennmaterial vom Typ $C_{18}$ gefüllt ist, chromatographiert, wobei als Eluenten (A) 0,1% Trifluoressigsäure in Wasser und (B) 0,1% Trifluoressigsäure in Acetonitril mit 40% (A) (v/v) dienen und isokratische Bedingungen (63% (A) + 37% (B)) herrschen und man vier Thrombin-hemmende Fraktionen erhält, von denen die erste Fraktion das gewünschte Hirudin-PA enthält;

H) zur Herstellung der desulfatierten Formen des Hirudin-PA die Sulfatmonoestergruppe am phenolischen Hydroxyl des Tyrosinrestes in Stellung 64, gewünschtenfalls auch die endständigen Aminosäuren D, E in Stellung 66, 65 einzeln oder gemeinsam hydrolytisch abspaltet, und

I) zur Herstellung der Abbauprodukte des Hirudin-PA dieses mit einer Peptidase inkubiert, die Produkte durch RP—HPLC auftrennt und die einzelnen Produkte isoliert und lyophilisiert.

3. Verfahren nach Anspruch 2, dadurch gekennzeichnet, daß man die Chromatographie in Stufe A an Sephadex G—75 mit einer Pufferlösung, die 50 mM Triethanolamin und 300 mM NaCl enthält, durchführt.

4. Verfahren nach Anspruch 2, dadurch gekennzeichnet, daß man die Anionenaustauschchromatographie in Stufe C an Whatman DE—52 oder DE—53 durchführt, wobei man mit einer Pufferlösung, die 30 mM Ammoniumacetat enthält, äquilibriert und mit einem Natriumacetatpuffer, der 200 mM Natriumacetat und 190 mM NaCl enthält, eluiert.

5. Verfahren nach Anspruch 2, dadurch gekennzeichnet, daß man die hydrolytische Abspaltung in Stufe H mit einer Arylsulfatase durchführt.

6. Verfahren nach Anspruch 2, dadurch gekennzeichnet, daß man als Peptidase in Stufe I Kathepsin C verwendet.

7. Verfahren nach Anspruch 2, dadurch gekennzeichnet, daß man Chromatographie in Stufe E an Sephadex DEAE—25 mit einem Natriumacetatpuffer, der 200 mM Natriumacetat und 190 mM NaCl enthält, durchführt.

8. Pharmazeutische Mittel, enthaltend mindestens eine Verbindung nach Anspruch 1, gegebenenfalls zusammen mit einem pharmazeutischen Träger oder Hilfsstoff.

9. Verwendung der Verbindungen nach Anspruch 1 zur Blutkonservierung, als Reagens zur analytischen Bestimmung von Thrombin oder zur Herstellung eines pharmazeutischen Mittels zur Hemmung der Blutgerinnung bei Mensch und Säugetier.

**Revendications**

1. Hirudine-PA de formule I:

```
                           10                  20
I T Y T D C T E S G Q N L C L C E G S N

                           30                  40
V C G K G N K C I L G S N G K D N Q C V

                           50                  60
T G E G T P K P Q S H N Q G D F E P I P

         *                    *
E D A Y D E                   Y=Tyrosin-O-Sulfat
```

dans laquelle les lettres symbolisent les constituants acides aminés selon la nomenclature IUPAC, qui sont liés par des liaisons peptidiques, et ses dérivés ayant jusqu'à deux acides aminées en moins du côté N-Terminal, ainsi que leur dérivés désulfatés, pour lesquels, dans la formule I ci-dessus:

i) le groupement ester sulfate sur l'hydroxyle phénolique du groupe tyrosine en position 64 est absent, ou

ii) le groupe ester sulfate spécifié sous i) et le dernier ou les deux derniers acides aminés D, E (en position 66 ou en positions 66 + 65) sont absents,

et les sels de ces composés utilisables en pharmacie.

2. Procédé de préparation de l'hirudine-PA et de ses dérivés tels que défini dans la revendication 1, dans lequel on broie des sangsues ou des extrémités de tête de sangsues, on homogénéise, on extrait l'homogénat de tissus avec un mélange acétone-eau ou avec une solution saline ou une solution tampon, que l'on précipite avec de l'éthanol, de façon fractionnée, une partie importante des impuretés de l'extrait, on concentre la solution sous vide, on soumet la solution concentrée à une précipitation fractionnée avec de l'acétone, on isole le précipité obtenu avec la concentration la plus élevée en acétone, on extrait le précipité à l'eau et on lyophilise l'extrait, caractérisé par le fait que;

A) l'on soumet le lyophilisat à une chromatographie sur colonne de Sephadex qui a été équilibrée avec un tampon de pH 7,8,

B) on lyophilise les fractions ayant une activité antithrombine, on dessale le lyophilisat et lyophilise à nouveau,

C) on soumet le Lyophilisat à une chromatographie d'échange d'anions sur DEAE-cellulose, on équilibre avec un tampon de pH 6,5 et on élue avec un tampon de pH 6,0,

D) on lyophilise les fractions ayant une activité antithrombine, dessale et lyophilise,

E) on fait passer le lyophilisat dans une colonne de DEAE-Séphadex qui a été équilibrée avec un tampon de pH 6,0, on élue avec une solution tampon ayant un gradient linéaire de pH qui est obtenue à partir d'un tampon de pH 5,0 et d'un tampon de pH 3,7,

F) on lyophilise les fractions ayant une activité antithrombine qui sont éluées à un pH de 4,6 à 4,7 environ, dessale et lyophilise à nouveau,

G) puis on chromatographie le lyophilisat sur une colonne pour HPLC, qui est remplie avec un matériau de séparation pour phase inversée du type $C_{18}$, en utilisant comme éluants (A) 0,1% d'acide

trifluoroacétique dans l'eau et (B) 0,1% d'acide trifluoroacétique dans l'acétonitrile avec 40% de (A) (v/v) et l'on procède dans des conditions isocratiques (63% (A) + 37% (B)) et l'on obtient quatre fractions inhibitrice de la thrombine, dont la première fraction contient l'Hirudine-PA désirée;

H) pour préparer les formes désulfatées de l'Hirudine-PA, on élimine par hydrolyse le groupe monoestersulfate de l'hydroxyle phénolique du reste tyrosine en position 64 et si désiré, l'un des acides terminaux D, E en position 66, 65 ou les deux,

I) pour préparer les produits de dégradation de l'hirudine-PA on incube celle-ci avec une peptidase, sépare les produits par RP—HPLC et isole les produits séparés et lyophilise.

3. Procédé selon la revendication 2, caractérisé par le fait que l'on effectue la chromatographie dans l'étape A sur Sephadex G—75 avec une solution tampon qui contient 50 mM de triéthanolamine et 300 mM de NaCl.

4. Procédé selon la revendication 2, caractérisé par le fait que l'on effectue la chromatographie d'échange d'anions dans l'étape C sur Whatman DE—52 ou DE—53, que l'on équilibre avec une solution tampon qui contient 30 mM d'acétate d'ammonium, et que l'on élue avec un tampon acétate de sodium qui contient 200 mM d'acétate de sodium et 190 mM de NaCl.

5. Procédé selon la revendication 2, caractérisé par le fait que l'on effectue l'hydrolyse dans l'étape H avec une arylsulfatase.

6. Procédé selon la revendication 2, caractérisé par le fait que l'on utilise comme peptidase dans l'étape I la Cathepsine C.

7. Procédé selon la revendication 2, caractérisé par le fait que l'on effectue la chromatographie dans l'étape E sur Sephadex DEAE—25 avec un tampon acétate de sodium qui contient 200 mM d'acétate de sodium et 190 mM de NaCl.

8. Composition pharmaceutique contenant au moins un composé selon la revendication 1, éventuellement en association avec un support pharmaceutique ou un adjuvant.

9. Utilisation des composés selon la revendication 1 dans la conservation du sang, comme réactif pour la détermination analytique de la thrombine ou dans la préparation d'une composition pharmaceutique pour inhiber la coagulation du sang chez l'homme et les mammifères.

**Claims**

1. Hirudin-PA of formula I:

```
                  10                  20
I T Y T D C T E S G Q N L C L C E G S N

                  30                  40
V C G K G N K C I L G S N G K D N Q C V

                  50                  60
T G E G T P K P Q S H N Q G D F E P I P

      *                *
E D A Y D E            Y=Tyrosin-O-Sulfat
```

in which the above letters represent proteinogenic amino acids corresponding to the IUPAC nomenclature, said amino acids being peptidically bonded, or derivatives thereof, shortened by up to two amino acids at the N-terminus as well as the desulfated derivatives thereof, wherein in the above formula I:

i) the sulfate ester group at the phenolic hydroxyl of the tyrosine group in position 64 is missing, or

ii) the sulfate ester group as described in i) and the latter or the two last amino acids D, E (in position 66 or 66 + 65) are missing,

and the pharmaceutically useful salts thereof.

2. A process for preparing hirudin-PA and the derivatives thereof as defined in claim 1, wherein leeches (Hirudo medicinalis) or the head ends of leeches are reduced, homogenized, the tissue paste is extracted with aqueous aceton or salt- or buffer-solution, a significant portion of the impurities of the extract is precipitated with ethanol during fractionation, the solution is reduced in a vacuum, the reduced solution is subjected to fractionating aceton precipitation, the precipitate obtained at higher aceton concentrations is isolated, extracted with water and the extract is lyophilised, characterized in that

A) the lyophilisate that is obtained is chromatographed on a Sephadex column that has been equilibrated with a buffer of pH 7,8;

B) the fractions with antithrombin activity are lyophilised, the lyophilisate is desalinated, and lyophilised once again;

C) an anion exchange chromatography is carried out on DEAE-celluloses with the lyophilisate, equilibration being carried out with a buffer of pH 6,5 and elution being carried out with a buffer of pH 6,0;

D) the fractions with antithrombin activity are lyophilised, desalinated, and lyophilised;

E) the lyophilisate is placed on a DEAE-Sephadex column that has been equilibrated with a buffer of pH 6,0, eluted with a buffer solution with a linear pH gradient that is formed of a buffer of pH 5,0 and a buffer of pH 3,7;

F) the fractions with antithrombin activity, eluted at approximately pH 4,6 to 4,7 are lyophilised, desalinated, and again lyophilised;

G) finally, the lyophilisate is chromatographed on a HPLC column that is filled with a reversed phase sequestering agent of the type $C_{18}$, with 0,1% trifluoroacetic acid in water serving as eluent (A) and 0,1% trifluoroacetic acid in acetonitrile with 40% (A) (v/v) serving as eluent (B), under isocratic conditions (63% (A) + 37% (B)), wherein one obtains four thrombin-suppressing fractions, with the first of these containing the desired hirudin-PA;

H) for preparation of the desulphated forms of hirudin-PA the sulfate monoester group of the phenolic hydroxyl of the tyrosin group in position 64, and if desired the terminal amino acids D, E in positions 66, 65 are split off hydrolytically, either separately or together; and

I) for preparing the reduction decomposition products of hirudin-PA this is incubated with a peptidase, the products are separated through RP—HPLC, and the individual products are isolated and lyophilised.

3. The process of claim 2, characterized in that the chromatography in stage A is carried out on Sephadex G—75 in a buffer solution with 50 mM triethanolamine and 300 mM NaCl.

4. The process of claim 2, characterized in that the anion exchange chromatography in stage C is carried out on Whatman DE—52 or DE—53 by equilibration with a buffer solution containing 30 mM ammonium acetate, and elution with a sodium acetate buffer containing 200 mM sodium acetate and 190 mM NaCl.

5. The process of claim 2, characterized in that the hydrolytical cleavage in stage H is carried out with an aryl sulfatase.

6. The process of claim 2, characterized in that the peptidase in stage I is kathepsin C.

7. The process of claim 2, characterized in that the chromatography in stage E is carried out on Sephadex DEAE—25 with a sodium acetate buffer containing 200 mM sodium acetate and 190 mM NaCl.

8. Pharmaceutical composition containing at least one compound as defined in claim 1, optionally in combination with a pharmaceutical carrier or adjuvant.

9. The use of a compound of claim 1 for the conservation of blood, as reagent for an analytical determination of thrombin or for the preparation of pharmaceutical compositions to inhibit the blood coagulation in man and mammalians.